# EUROPÄISCHE PATENTSCHRIFT

(11) **EP 3 464 530 B1**
(45) Veröffentlichungstag und Bekanntmachung des Hinweises auf die Patenterteilung: **27.08.2025**
(21) Anmeldenummer: 17727828.0
(22) Anmeldetag: 23.05.2017
(51) Int. Cl.: A61L 9/012, C11C 5/00, F21V 35/00, A61L 9/03, C06F 3/04, A01M 1/20, A61L 9/02, A61L 9/04, F21V 37/00, A61L 9/12

(54) **KERZE MIT EINEM KOMPOSTIERBAREN KUNSTSTOFFELEMENT**
CANDLE COMPRISING A COMPOSTABLE PLASTIC ELEMENT
BOUGIE MUNIE D'UN ÉLÉMENT EN PLASTIQUE COMPOSTABLE

(30) Priorität: 24.05.2016 DE 102016208993; 07.03.2017 DE 102017203743
(43) Veröffentlichungstag der Anmeldung: 10.04.2019
(73) Patentinhaber: Cup Candle GmbH, 19258 Greven (DE)
(72) Erfinder: INDERBIETHEN, Karsten, 21521 Wohltorf (DE)
(74) Vertreter: Kutzenberger Wolff & Partner
(86) Internationale Anmeldenummer: PCT/EP2017/062468
(87) Internationale Veröffentlichungsnummer: WO 2017/202868

(56) Entgegenhaltungen:
- EP-A1- 1 932 545
- WO-A1-00/56846
- WO-A1-2016/128490
- WO-A1-97/30138
- DE-A1- 2 943 662
- DE-A1- 4 242 509
- DE-U1- 202012 102 360
- DE-U1- 9 205 565
- DE-U1- 9 208 744
- US-A- 2 137 707
- US-A1- 2003 134 244
- US-A1- 2015 056 562
- US-A1- 2015 374 869
- US-E- R E20 434
- XIAOMIN ZHAO ET AL: "New Superefficiently Flame-Retardant Bioplastic Poly(lactic acid): Flammability, Thermal Decomposition Behavior, and Tensile Properties", ACS SUSTAINABLE CHEMISTRY & ENGINEERING, vol. 4, no. 1, 11 December 2015 (2015-12-11), US, pages 202 - 209, XP055433150, ISSN: 2168-0485, DOI: 10.1021/acssuschemeng.5b00980
- DOMINIQUE HELLIO ET AL: "Chemically and Physically Cross-linked Gelatin Gels", 1 January 2003 (2003-01-01), XP055402242, Retrieved from the Internet <URL:http://www.issp.u-tokyo.ac.jp/public/GelSympo2003/Proceedings/digitalproceedings/pdf_files/II_05Djabourov.pdf> [retrieved on 20170830]
- DIETER KLEMM ET AL: "Cellulose: Fascinating Biopolymer and Sustainable Raw Material", ANGEWANDTE CHEMIE INTERNATIONAL EDITION, vol. 44, no. 22, 30 May 2005 (2005-05-30), Hoboken, USA, pages 3358 - 3393, XP055433112, ISSN: 1433-7851, DOI: 10.1002/anie.200460587
- ANONYMOUS: "Illatos, természetes és sokoldalú alapanyag: ötletek narancshéjból", 25 September 2013 (2013-09-25), XP055401825, Retrieved from the Internet <URL:http://szinesotletek.blog.hu/2013/09/25/illatos_termeszetes_es_sokoldalu_alapanyag_otletek_narancshejbol> [retrieved on 20170829]
- ANONYMOUS: "Biokunststoff", 7 August 2017 (2017-08-07), XP055402226, Retrieved from the Internet <URL:https://de.wikipedia.org/wiki/Biokunststoff> [retrieved on 20170830]

## Beschreibung

Die vorliegende Erfindung betrifft betrifft die Erfindung ein Verfahren zur Herstellung einer Kerze aufweisend ein brennbares Material und einen Docht und ein becherartiges Gefäß , wobei das becherartige Gefäß zumindest teilweise aus einem Biopolymer basieren auf Gelatine gebildet wird, dadurch gekennzeichnet, dass eine Form des herzustellenden becherartigen Gefäßes ein- oder mehrmals in das flüssige Biopolymer basierend auf Gelatine eingetaucht wird, bis eine gewünschte Materialstärke erreicht wird.

Derzeit werden Kerzen, insbesondere Teelichter, in einem Aluminiumbehälter oder in einem spritzgegossenen Polycarbonatbehälter vorgesehen. Die Herstellung dieser Kerzen ist jedoch sehr aufwändig und es entsteht eine große Menge an umweltbelastendem Verpackungsmüll. Dieses Problem ergibt sich insbesondere in Kirchen, in Tempeln sowie auf Friedhöfen, wo Kerzen in großer Anzahl verwendet werden. Ein weiteres Anwendungsbeispiel sind Schwimmkerzen, die religiösen Bräuchen folgend auf Wasserflächen abgebrannt werden. Es kann diesbezüglich auf DE 20 2012 102360 U1, DE 42 42 509 A1, US 2003/0134244 A1, US 2015/056562 A1, US E20 434 E1, WO 00/56846 A1 und WO 2016/128490 A1 verwiesen werden.

Es war deshalb die Aufgabe der vorliegenden Erfindung, eine Kerze zur Verfügung zu stellen, die die Nachteile des Standes der Technik nicht aufweist.

Gelöst wird diese Aufgabe durch das Verfahren gemäß Patentanspruch 1.

Beschrieben wird eine Kerze aufweisend ein brennbares Material und einen Docht und ein Kunststoffelement, wobei das Kunststoffelement zumindest teilweise oder vollständig aus einem Biopolymer, insbesondere Gelatine und/oder Polysacharid und/oder Cellulose, besteht.

Beschrieben wird ferner ein Verfahren zur Herstellung einer Kerze aufweisend ein brennbares Material und einen Docht und ein Kunststoffelement, wobei das Kunststoffelement zumindest teilweise oder vollständig aus einem Biopolymer gebildet wird. Die vorliegende Beschreibung betrifft eine Kerze, die einen Docht, ein brennbares Material und ein Kunststoffelement aufweist. Das Kunststoffelement weist vorzugsweise einen Boden- und einen Wandbereich auf. Der Boden- und Wandbereich sind vorzugsweise einstückig vorgesehen, können alternativ dazu aber auch mehrteilig vorgesehen sein, wobei der Boden- und Wandbereich miteinander verbunden, insbesondere miteinander verklebt, verschachtelt oder kraftschlüssig verbunden, sind. Erfindungsgemäß handelt es sich bei dem Kunststoffelement um ein becherartiges Gefäß, das das brennbare Material zumindest teilweise, vorzugsweise vollständig aufnimmt. Das Kunststoffelement kann als Mulde ausgebildet sein. Der äußere Umfang des Kunststoffelements, insbesondere der Wandbereich kann aber auch zumindest teilweise von dem brennbaren Material umschlossen werden. Für den Abbrand der Kerze wird das Kunststoffelement nicht von dem brennbaren Material getrennt, sondern stellt ein wichtiges Element der Kerze dar, beispielsweise um zu verhindern, dass das brennbare Material unkontrolliert verläuft und/oder beispielsweise zum Brandschutz, beispielsweise damit der Docht nicht in Kontakt mit der Auflagefläche der Kerze kommt. Gemäß einer weiteren bevorzugten Ausführungsform ist das Kunststoffelement ein Dochthalter. Der Dochthalter kann mit dem Docht, insbesondere mit einem Ende des Dochts, verbunden sein. Besonders bevorzugt ist der Dochthalter mit dem Boden eines becherartigen Kunststoffelementes oder eines becherartigen Metallelements verbunden.

Das brennbare Material ist insbesondere Wachs, beispielsweise Bienenwachs, Stearin und/oder Paraffin, also insbesondere gehärtete oder raffinierte pflanzliche und/oder tierische Fette, wachsähnliche Materialien und/oder technische Wachsgemenge. Der Docht wird von dem brennbaren Material umgeben. Das brennbare Material ist zunächst fest und wird durch die Wärmeabgabe des brennenden Dochts zumindest teilweise verflüssigt. Das brennbare Material weist vorzugsweise keinerlei Farbstoffe auf. Vorzugsweise ist das brennbare Material mit einem oder mehreren Duftstoffen versetzt. Vorzugsweise ist das brennbare Material mit einem Zusatz versetzt, der eine Flammfärbung bewirkt. Hierdurch ist es in vorteilhafter Weise möglich, eine weiße oder farbige Kerze bereitzustellen, insbesondere auch eine Kerze, die auch im angezündeten Zustand farbig ist, d.h. eine gefärbte Flamme aufweist. Falls auch das Material, aus dem die Mulde bzw. das Material gefertigt ist, farbig ist, wird ein ästhetisch ansprechender Gesamtfarbeindruck erzielt.

Sofern die Kerze geschlossen ausgebildet ist, können auch bei Raumtemperatur, insbesondere bei 14 bis 26°C, flüssige Brennmaterialien eingesetzt werden.

Grundsätzlich kommen alle üblichen, dem Fachmann bekannten Brennmaterialien, wie Lampenöle beispielsweise auf Basis von Mineralöl, Petroleum oder Paraffin in Betracht.

Bevorzugt werden als flüssige Brennmaterialien pflanzliche Öle, vorzugsweise Rapsöl, Sonnenblumenöl, Olivenöl oder Mischungen aus wenigstens zwei dieser vorgenannten Materialien eingesetzt.

Diese flüssigen Brennmaterialien können farblos sein oder einen oder mehrere Farbstoffe aufweisen, um so einen ästhetisch ansprechenden Eindruck vermitteln. Vorzugsweise kann das flüssige Brennmaterial mit einem oder mehreren Duftstoffen versetzt sein. Vorzugsweise kann das flüssige Brennmaterial mit einem Zusatz versetzt sein, der eine Flammfärbung bewirkt. Hierdurch ist es in vorteilhafter Weise möglich, eine weiße oder farbige Kerze bereitzustellen, insbesondere auch eine Kerze, die auch im angezündeten Zustand farbig ist, d.h. eine gefärbte Flamme aufweist. Das Material, aus dem die Mulde bzw. das Material gefertigt ist, kann ebenfalls farbig sein.

Das flüssige Brennmaterial weist vorzugsweise einen Duftstoff auf, dessen Duft sich beim Abrennen des Brennmaterials entfaltet und/oder ändert. Alternativ oder zusätzlich weist das flüssige Brennmaterial einen Farbstoff auf, der sich beim Abrennen des Brennmaterials verteilt, aktiviert wird oder den farblichen Gesamteindruck verändert.

Die flüssigen Brennmaterialien sind wesentlich kostengünstiger als beispielsweise gehärtete, raffinierte Wachse aus Mineralöl oder Pflanzenfette. Ein weiterer Vorteil, insbesondere der pflanzlichen Öle, besteht darin, dass diese aus nachwachsenden Rohstoffen erhalten werden, und sehr rein, insbesondere rußfrei, abrennen. Durch die Verwendung flüssiger Brennmaterialien können gegenüber der Verwendung fester Brennmaterialien einige Verarbeitungsschritte bei deren Herstellung entfallen, so dass diese Zeit- und Energiesparender hergestellt werden können.

Ein weiterer großer Vorteil, den der Einsatz von flüssigen Brennmaterialien mit sich bringt, besteht darin, dass die Kerzen enthaltend diese Brennmaterialien auch bei kälteren Temperaturen abgebrannt werden können, als Kerzen, die feste Brennmaterialien aufweisen, beispielsweise bei Temperaturen von bis zu -25°C oder bis zu -10°C oder bis zu - 5°C oder bis zu 0°C. Dies ist besonders vorteilhaft bei allen Anwendungen, bei denen die Kerze unter kühlen Außenbedingungen oder in kühlen Räumen abgebrannt wird, beispielsweise bei der Anwendung als Schwimmkerze, Friedhofskerze, Kirchen oder Tempeln.

Erfindungsgemäß besteht das Kunstoffelement ganz oder zumindest teilweise aus einem Biopolymer, basierend auf Gelatine. Erfindungsgemäß können auch Mischungen von zwei oder mehreren Biopolymeren eingesetzt werden, wobei auch Mischungen von zwei oder mehr Biopolymeren unterschiedlicher Klassen beispielsweise Gelatine und Polysacharide oder Gelatine und Cellulose oder auch Gelatine und Polysaccharide und Cellulose in Betracht kommen. Diese Materialien haben den weiteren Vorteil, dass die daraus erhaltenen Kunststoffelemente vegan und/oder koscher und/oder halal hergestellt werden können.

Erfindungsgemäss besteht das Kunstoffelement zumindest teilweise aus Gelatine oder Mischungen aus zwei oder mehr dieser Biopolymere. Vorzugsweise beträgt der Biopolymeranteil in dem Material, aus dem das Kunstoffelement hergestellt wird, > 70 Gew.-%, vorzugsweise > 80 Gew.-%, noch mehr bevorzugt > 90 Gew.-% und am meisten bevorzugt > 95 Gew.-%. Insbesondere kann das Kunstoffelement auch vollständig aus Biopolymer bestehen.

Erfindungsgemäß noch weiter bevorzugt besteht das Kunststoffelement zumindest teilweise aus Gelatine und Polysacharid und/oder Cellulose. Vorzugsweise beträgt der Gelatine und Polysacharid- und/oder Celluloseanteil des Materials, aus dem das Kunststoffelement hergestellt wird > 70 Gew.-%, vorzugsweise > 80 Gew.-%, noch mehr bevorzugt > 90 Gew.-% und am meisten bevorzugt > 95 Gew.-%. Insbesondere kann das Kunstoffelement auch vollständig aus Biopolymer bestehen.

Erfindungsgemäß am meisten bevorzugt besteht das Kunststoffelement zumindest teilweise aus Gelatine und Hydroxypropylmethylcellulose und/oder Pullulan. Vorzugsweise beträgt der Gelatine- und Hydroxypropylmethylcellulose- und/oder Pullulananteil des Materials, aus dem das Kunststoffelement hergestellt wird > 70 Gew.-%, vorzugsweise > 80 Gew.-%, noch mehr bevorzugt > 90 Gew.-% und am meisten bevorzugt > 95 Gew.-%. Insbesondere kann das Kunstoffelement auch vollständig aus Biopolymer bestehen.

Gelatine ist vorzugsweise ein tierisches Protein, vorzugsweise ein Stoffgemisch. Hauptbestandteil ist vorzugsweise denaturiertes bzw. hydrolysiertes Kollagen, das aus dem Bindegewebe verschiedener Tierarten, vor allem Schweinen und Rindern, aber auch Fischen und Geflügel produziert werden kann. Bei der Gelatine kann es sich aber auch um pflanzliche Gelatine handeln, die meistens Polysacharide sind.

Als Gelatine kommt erfindungsgemäß insbesondere Gelatine zum Einsatz, wie sie im Bereich der pharmazeutischen Technologie zur Herstellung von Hartgelatine oder Weichgelatinekapseln Verwendung findet.

Vorzugsweise weist die Gelatine im Kunststoffprodukt einen Wassergehalt von 6-20 Gew.%, noch mehr bevorzugt von 9-18 Gew.-%, viel mehr bevorzugt 10-16 Gew.-% und am meisten bevorzugt 12-16 Gew.-% auf.

Vorzugsweise kann die Gelatine, wie sie zur Herstellung des Kunstoffproduktes eingesetzt wird, einen Bloom-Wert von 50-300, besonders bevorzugt von 150-280 Bloom oder 180-280 aufweisen.

Vorzugsweise ist die Gelatine gehärtet und/oder wasserunlöslich gemacht. Alternativ dazu ist die Gelatine gehärtet und/oder temporär wasserunlöslich oder temporär feuchtigkeitsresistent. Unter einer temporär wasserunlöslichen oder temporär feuchtigkeitsresistenten Gelatine wird beispielsweise Gelatine verstanden, die nach einer gewissen Zeit, beispielsweise nach 1-14 Tagen, insbesondere 1-7 Tagen, bevorzugt 1-5 Tagen, Anzeichen einer Zersetzung zeigt.

Dies ist beispielsweise durch Vernetzung des Produktes mit einer oder mehreren Chemikalien erreichbar, also inbsesondere ein Prozess, der das Molekulargewicht des Proteins wirksam erhöht. Dafür eignen sich beispielsweise Alginsäure-Alkylenglykolderivate, mit Natriumperoxid oxidierte Alginsäure, Amylopectindialdehydoktenylsuccinat, Bisacryloylurea, Bis(azidinethyl)sulfon, Bis(bromacetyl)ethylendiamin, Bis(chlormethyl)adipat, Bis(chlormethyl)diethylmalonat, Bis(chloracetyl)dimethylendiamin, Benzoldisulfonylfluorid, Bromessigsäure, Carbodiimide, Carboxylbenzylbromid, Bis(chlorethyl)urea, Chromacetat und -alaune, Cyallurchlorid, Dialdehydstärke behandelt mit kaustischer Soda, Dichlorhydroxy-s-triazin, Natriumsalz, Dichlorquinoxolinkarbonylchlorid, Diethylenurea, Diformyldihydroxytrioxanonan, Ditrioxynaphthalin, Difluordinitrobenzol, Dihydroxydioxan, Diglycidylmono(propylenchlorhydrin)ether von Glycerin, Dihydroxymaleillsäure, Di(maleimido)benzol, Di(maleimido-)hexan, Dimaleimid, Dimethylaminophosphoryl, Bis(dimethylamino)phosphorylchlorid, Dimethylbis(vinylsulfonyl)benzol, Dinatriumsalz, Tris(sulfatetyl)sulfonium-Innerethersalze, Ethylenglykol, Ethoxymethylisocyanat, Formalin(formaldehyd, Fluorsulfonylazetophenon, Glycerin, Gummi arabicum, beispielsweise oxidiert mit Perjodsäure, Glutaraldehyd, Hydroxytetramethylhydrooxazoniumchlorid, Milchsäure, Methotoluolsulfonat, Mono-I-O-bromethylmaleat, Methylendiethansulfonamid, Methansulfonsäurehalogenalkoholester, Mercaptoethylaziridin, Methylglucanopyranosid, Naphthalindisulforylfluorid, Pektinsäure, Peroxydisulfat, Phenolformaldehydharze, Phenyltriazinidinylsilan, Phloroformalin, Phosphonitrilchlorid, Phloroglucin, Polyurethan, Polyformal(e), Polyacrolein, Polyvinylmaleat, Kaliumpermanganat, Kaliumferrocyanid, Pyromellithanhydrid, Resorcindiglycidylether, Resorcin, Saccharid(e), Sulfanilinodichloro-s-triazin, Natriumhypochlorit, Natriumsalz von Dichlorhydroxytriazin, Stearylaminobenzoyl-Essigsäuure, Sucrose, oxidiert mit Perjodat,Tannin, Tetrahydroxyphenol, Trimellithanhydrid, Trimethylenbis(isomaleimid), Tris(chloracetyl)hexahydrotriazin, Tetraisopropyltitanat und/oder deren Gemisch aus mindestens zwei der oben genannten Substanzen.

Polysacharid ist der Hauptbestandteil von pflanzlichen Zellwänden. Vorzugsweise wird das Polysacharid in Form von Cellulose, beispielsweise Methyl-Cellulose und/oder Hydroxypropylmethyl-Cellulose und/oder Celluloseacetat, vorgesehen. Andere Cellulose basierende Verbindungen sind ebenfalls denkbar. Alternativ kann das Polysacharid in Form von Pullulan vorgesehen. Das Polysacharid kann als Mischung von mehreren Substanzen zur Verfügung gestellt werden. Weiterhin kann das Kunststoffelement ganz oder teilweise aus Cellulose, beispielsweise Methyl-Cellulose und/oder Hydroxypropylmethyl-Cellulose und/oder Celluloseacetat bestehen. Andere Cellulose basierende Verbindungen sind ebenfalls denkbar.

Unter Cellulosen werden auch deren Derivate verstanden, inbesondere Celluloseether, welche durch teilweise oder vollständige Substitution der Wasserstoff-Atome der Hydroxy-Gruppen in der Cellulose entstehen. Diese Celluloseether können vorzugsweise ausgewählt werden aus der Gruppe bestehend aus Carboxymethylcellulose, Methylcellulose, Ethylcellulose, Hydroxyethylcellulose, Hydroxypropylcellulose, Methylethylcellulose, Hydroxyethylmethylcellulose, Hydroxypropylmethylcellulose, Ethylhydroxyethylcellulose und Carboxymethylhydroxyethylcellulose. Erfindungsgemäß können dabei auch Mischungen aus zwei oder mehr dieser Celluloseethyer zum Einsatz kommen.

Ganz besonders bevorzugt ist Hydroxypropylmethylcellulose. Eine entsprechend geeignete Hydroxypropylmethylcellulose wird beispielsweise auch in der Herstellung von veganen Hartkapseln im Bereich der pharmazeutischen Technologie eingesetzt.

Vorzugsweise weist die Hydroxypropylmethylcellulose im Kunststoffprodukt einen Wassergehalt von 1-23 Gew.%, noch mehr bevorzugt von 1,5-20 Gew.-%, viel mehr bevorzugt von 2,0-15 Gew.-% und am meisten bevorzugt 2,5-10 Gew.-% auf.

Erfindungsgemäß wird im folgenden unter der Bezeichnung Kunststoff basierend auf Gelatine und/oder Polysaccharid und/oder Cellulose, insbesondere Kunststoff basierend auf Gelatine und/oder Hydroxypropylmethylcellulose und/oder Pullulan verstanden. Erfindungsgemäß wird eine Form des herzustellenden Kunststoffelementes ein- oder mehrmals in ein flüssiges Biopolymer, basierend auf Gelatine insbesondere eine flüssige Kunststoffmasse, eingetaucht bis die gewünschte Materialstärke erreicht wird.

Sodann erstarrt das Kunststoffmaterial beispielsweise mittels Trocknung, Wärme- und/oder Kältebehandlung und/oder Vernetzung, beispielsweise mittels IV- oder UV-Strahlung, bis das Biopolymer, insbesondere der Kunststoff, so weit erstarrt ist, dass er die Verwendung als Kunststoffelement für Kerzen zulässt. Durch mehrmaliges Eintauchen der Form kann ein Kunststoffelement mit mehreren Schichten erzeugt werden. Die Form kann nacheinander in verschiedene flüssige Biopolymere eingetaucht werden. Die Schichten, die durch das Eintauchen in verschiedene flüssige Biopolymere erzeugt werden, können sich hinsichtlich ihrer optischen Eigenschaften, ihrer UV-Beständigkeit, Härte, Entflammbarkeit, und/oder Dichtheit gegenüber Flüssigkeiten unterscheiden.

In diesem Zusammenhang hat es sich als vorteilhaft erwiesen, wenn die Form eine Tauchform des Kunststoffelements ist, wobei die Tauchform eine Außenkontur mit einem Tauchbereich aufweist, welcher in das flüssige Biopolymer eingetaucht wird. Der Tauchbereich entspricht bevorzugt einem Negativ der Innenkontur des herzustellenden Kunststoffelements. Bei dem Herstellungsverfahren wird der Tauchbereich der Außenkontur mit einer Schicht des Biopolymers überzogen. In einem weiteren Schritt kann die Form aus dem flüssigen Biopolymer entnommen werden. Die Schicht des Biopolymers, die die Außenkontur der Form überzieht, kann erstarren und getrocknet werden. Ein entsprechendes Verfahren ist beispielsweise im Bereich der Pharmazie als Colton-Verfahren zur Herstellung von pharmazeutischen Kapseln bekannt. Insbesondere kann die überzogene Form während des Erstarrens und/oder Trocknens in einer oder mehreren Dimensionen gesteuert werden, um die Ausgestaltung des Kunststoffelements, insbesondere die Form und/oder die Dicke gezielt einzustellen.

Das erstarrte Kunststoffelement kann dann mit dem brennbaren Material und/oder dem Docht verbunden werden.

Gemäß einer vorteilhaften Ausgestaltung des erfindungsgemäßen Verfahrens ist vorgesehen, dass die Form durch das brennbare Material der Kerze gebildet wird. Das brennbare Material kann ein- oder mehrmals in das flüssige Biopolymer eingetaucht werden. Das brennbare Material kann teilweise in das flüssige Biopolymer eingetaucht werden. Auf diese Weise lässt sich ein becherartiges Gefäß aus dem Biopolymer erzeugen, dessen Innenkontur an die Außenkontur des brennbaren Materials angepasst ist. Alternativ ist es möglich, dass brennbare Material vollständig in das flüssige Biopolymer einzutauchen, so dass ein verkapseltes Behältnis für das brennbare Material gebildet wird. Bevorzugt ist der Docht der Kerze beim Eintauchen des brennbaren Materials in das flüssige Biopolymer bereits mit dem brennbaren Material verbunden. Besonders bevorzugt wird das brennbare Material beim Eintauchen durch den Docht gehalten, so dass das brennbare Material mittels dem Docht aus dem flüssigen Biopolymer herausgezogen werden kann.

Vorzugsweise ist das Material, aus dem das Kunststoffelement hergestellt wird, nicht brennbar und/oder schwer entflammbar. Trotz dieser nicht brennbaren bzw. schwer entflammbaren Eigenschaften ist das Kunststoffelement insbesondere weiterhin biologisch abbaubar. Besonders bevorzugt entspricht das Material, aus dem das Kunststoffelement hergestellt ist, einer Brandschutzklassifizierung gemäß der Norm UL94 V0, V1 oder V2 und/oder gemäß den Normen IEC/DIN EN 60 695-11-10 bzw. IEC/DIN EN 60 695-11-20. Dafür weist das Biopolymer, insbesondere die Gelatine und/oder das Polysacharid und/oder die Cellulose vorzugsweise eine brandhemmende Substanz auf, das ihr eine höhere Brandschutzklasse verleiht. Diese Substanz kann fest oder in flüssiger Form vorgesehen sein. Die Substanz kann mit der Gelatine und Polysacharid und/oder der Cellulose chemisch und/oder physikalisch gebunden und/oder beispielsweise wie eine Mikrokapsel von der Gelatine und Polysacharid und/oder Cellulose umschlossen sein.

Das Kunststoffelement aus Gelatine und Polysacharid und/oder Cellulose kann bei Raumtemperatur fest, das heißt elastisch, allenfalls nur geringfügig deformierbar, sein.

Alternativ ist das Kunststoffelement elastisch deformierbar, beispielsweise gelartig vorgesehen.

Gemäß einem bevorzugten Beispiel wird das Kunststoffelement aus einem Polysacharid, das unter dem Namen Pullulan geführt wird, hergestellt. Vorzugsweise weist die Komposition aus der das Kunststoffelement hergestellt wird folgende Bestandteile auf:
Pullulan ( FCC & E-1204 ) und Wasser; vorzugsweise Additive: Titaniumdioxid, Flammschutzmittel, UV Protector, vorzugsweise Verarbeitungshilfen: Sodium Lauryl Sulphat, pflanzliches Öl, Lubricant, vorzugsweise Hilfsstoffe wie Carrageenan, Lubricant, Sodium Lauryl Sulphat, Gellan, Xanthan und/oder Kaliumchlorid auf.

Das Kunststoffelement weist Gelatine auf. Vorzugsweise weist die Komposition aus der das Kunststoffelement hergestellt wird folgende Bestandteile auf:
Gelantine pflanzlich und/oder tierisch und Wasser, vorzugsweise Additve: Titaniumdioxid ( E - 171 ), Flammschutzmittel, UV Protector, vorzugsweise Verarbeitungshilfen: Sodium Lauryl Sulphat, Essigsäure , pflanzliches Öl.

Weiterhin bevorzugt weist das Kunststoffelement Cellulose auf. Vorzugsweise weist die Komposition aus der das Kunststoffelement hergestellt wird folgende Bestandteile auf: Hydroxypropylmethylcellulose (HPMC, E-464 & USP) und Wasser, vorzugsweise Additive: Titanium Dioxid ( E - 171 ), Flammschutzmittel, UV Protector, vorzugsweise Hilfsmittel: Careageenan ( E-407 & USP), Kaliumchlorid, vorzugsweise Verarbeitungshilfen, Citric Acid Estersof Mono-Diglycerides from refined Sunflower oil d.h. Zitronensäureester von Monodiglyceriden aus rafinierrtem Sonnenblumenöl auf.

Das Kunststoffelement kann aus einem Gemisch aus einer oder mehreren Gelatinesubstanzen und einem oder mehreren Polysachariden und/oder mehreren Celluloseverbindungen vorgesehen sein.

Der Querschnitt des Kunststoffelementes ist vorzugsweise so vorgesehen, dass die Länge des Dochts, der aus dem brennbaren Material heraussteht, immer kürzer ist als die kürzeste Dimension des Querschnitts der Mulde. Dadurch wird verhindert, dass der Docht oder die Flamme des Dochts mit einer Seitenwand der Mulde in Kontakt gerät.

Das Kunststoffelement kann transparent und/oder farbig sein. Das Material kann mit jedem beliebigen, dem Fachmann geläufigen Verfahren bedruckt werden. Insbesondere kann das Material transparent sein und farbig bedruckt werden. Hierdurch ist es vorteilhafterweise möglich, eine im Wesentlichen transparentes Kunststoffelement bereitzustellen und dennoch wichtige Produktinformationen und/oder Warnhinweise darauf vorzusehen. Ferner ist es im Falle eines wenigstens teilweise farbigen Materials vorteilhafterweise möglich, dass ein ansprechendes Erscheinungsbild der Kerze realisiert wird. Insbesondere für Duftkerzen ist somit eine einfache Farbkodierung realisierbar. So können zum Beispiel rote Kerzen, d.h. Kerzen, deren Kunststoffelement aus einem wenigstens teilweise roten Material hergestellt sind, nach Rosen duften, grüne Kerzen nach Apfel und weiße bzw. farblose Kerzenmagazine können im Wesentlichen duftlos sein. Bei transparenten Kunststoffelementen ergibt sich der weitere Vorteil, dass das Brennmaterial von außen sichtbar ist. Dies ermöglicht es den farblichen Gesamteindruck über die Farbe des Brennmaterials zu beeinflussen. Weiterhin kann von außen erkannt werden, ob das Brennmaterial flüssig oder fest ist.

Gemäß einer bevorzugten Ausführungsform weist das brennbare Material wenigstens teilweise die gleiche Farbe auf wie das Material, aus dem das Kunststoffelement hergestellt wird. Hierdurch ist es vorteilhafterweise möglich, ein einheitliches Erscheinungsbild der Kerze bereitzustellen.

Das zumindest teilweise, vorzugsweise vollständig aus Gelatine und Polysacharid und/oder Cellulose hergestellte Kunststoffelement hat den Vorteil, dass es kompostierbar ist. Erfindungsgemäß wird deshalb eine Kerze, beispielsweise ein Teelicht, zur Verfügung gestellt, das rückstandsfrei entsorgbar ist. Nach dem Abbrand der Kerze kann das Kunststoffelement in dem "Biomüll" entsorgt werden.

Gemäß einer bevorzugten Ausführungsform ist der Docht ein fadenförmiges Geflecht. Besonders bevorzugt weist der Docht, insbesondere an seiner Außenseite, ein Versteifungsmittel auf, beispielsweise einen Hartwachsüberzug und/oder eine Hülse, insbesondere aus einem brennbaren Material. Dadurch ist es vorteilhafterweise möglich, dass der Docht eine gewisse Eigenstabilität aufweist und/oder seine Brenneigenschaften in gewünschter Weise beeinflusst werden können.

Gemäß einer bevorzugten Ausführung weist der Docht in einem Brennstoppbereich ein Biopolymer, insbesondere Gelatine und/oder Polysacharid und/oder Cellulose, besonders bevorzugt Gelatine und/oder Hydroxypropylmethylcellulose und/oder Pullulan auf, so dass eine Kerze mit einem selbstlöschenden Docht bereitgestellt werden kann. Beispielsweise kann der Docht in dem Brennstoppbereich mit dem Biopolymer getränkt sein.

Gemäß einer bevorzugten Ausführungsform wird der Docht der Kerze direkt oder indirekt form-, kraft- und/oder stoffschlüssig mit dem Kunststoffelement verbunden. Beispielsweise weist der Docht einen Dochthalter auf, der vorzugsweise mit der Mulde durch Kleben, Siegeln, Schweißen oder vorzugsweise durch einen Schnappverschluss verbunden wird. Es ist aber auch denkbar, dass der Docht ohne einen Dochthalter form-, kraft- und/oder stoffschlüssig mit der Mulde verbunden wird, beispielsweise durch Einpressen und/oder Siegeln. Besonders bevorzugt werden der Docht, der Dochthalter und/oder die Mulde über Ultraschallsiegeln und/oder über einen, insbesondere feuerfesten, Klebstoff, insbesondere einen Schmelzklebstoff (Hotmelt) verbunden. Für den Fall, dass der Docht einen Halter aufweist, ist dieser vorzugsweise aus einem Werkstoff hergestellt, der Gelatine und/oder Polysacharid zumindest teilweise aufweist.

Gemäß einer bevorzugten Ausführung des erfindungsgemäßen Verfahrens wird ein Kunststoffelement gebildet, welches eine Mulde zur Aufnahme des brennbaren Materials und einen Dochthalter zum Halten des Dochts aufweist. Insofern sind Dochthalter und Mulde einstückig miteinander verbunden.

Gemäß einer bevorzugten Ausführungsform der vorliegenden Erfindung weist das Kunststoffelement einen Bodenbereich auf, in dem eine Ein- und/oder Ausbuchtung und/oder eine Öffnung vorgesehen ist, in der der Docht und/oder ein Dochthalter befestigt oder zumindest teilweise eingebettet ist. Die Befestigung erfolgt besonders bevorzugt durch einen Form-, Kraft- und/oder Stoffschluss. Durch die Ein- oder Ausbuchtung wird verhindert, dass der Docht bei der Herstellung des Kerzenmagazins und/oder beim Abbrand der Kerze verrutscht. Vorzugsweise weist die Ein- und/oder Ausbuchtung einen Hinterschnitt auf, der form- und/oder kraftschlüssig mit dem Docht und/oder mit dem Dochthalter zusammenwirkt.

Weiterhin bevorzugt ist der Bodenbereich zumindest abschnittsweise geneigt gegenüber der Horizontalen vorgesehen. Dadurch wird sichergestellt, dass das brennbare Material, das sich beim Abbrand der Kerze verflüssigt, in Richtung des Dochts fließt, so dass zumindest ein möglichst vollständiger Abbrand des brennbaren Materials sichergestellt ist.

Gemäß einer bevorzugten Ausführungsform ist die Seitenwand des Kunststoffelements zumindest abschnittsweise gegenüber der Vertikalen geneigt vorgesehen. Besonders bevorzugt weist die Mulde eine im Wesentlichen konische Form auf, wobei insbesondere der Durchmesser im Bodenbereich kleiner ist als der Durchmesser im Bereich einer dem Bodenbereich gegenüberliegenden Öffnung der Mulde. Hierdurch wird zum einen ein kontrollierter und vollständiger Abbrand des brennbaren Materials sichergestellt und zum anderen wird in besonders vorteilhafter Weise sichergestellt, dass die Kerze in Gefäße unterschiedlicher Passformen, d.h. unterschiedlicher Durchmesser, passt. Somit ist die Kerze vorteilhafterweise flexibel einsetzbar. Außerdem kann eine derartige Mulde besser entformt werden. Gemäß einer anderen bevorzugten Ausführungsform ist die Seitenwand des Kunststoffelements zylindrisch vorgesehen. Vorzugsweise ist Übergang zwischen der Seitenwand und dem Bodenbereich mit einem Krümmungsradius, besonders bevorzugt zwischen 1 und 5 mm vorgesehen. An dem dem Bodenbereich gegenüberliegenden Ende der Seitenwand kann eine Stapelkante vorgesehen sein, die es ermöglicht mehrere Kerzen übereinander zu Stapeln. In diesem Fall ist es vorteilhaft, wenn der Füllstand des brennbaren Materials und/oder der Docht so vorgesehen werden, dass der Docht die obere Kante der Seitenwand nicht überragt.

Vorzugsweise weist die Seitenwand des Kunststoffelements an seinem dem Bodenbereich gegenüberliegenden Ende einen im Wesentlichen horizontalen, vorzugsweise um den Umfang der Seitenwand umlaufenden, Flansch auf, der diesem Teil des Kunststoffelementes Stabilität verleiht und/oder als An und/oder Auflagefläche für ein Werkzeug, die Hände eines Menschen und/oder eines Gegenstands, in dem die Kerze abgebrannt wird, dienen kann.

Gemäß einer bevorzugten Ausführungsform weist das Kunststoffelement ein Mittel auf, das das brennbare Material form- und/oder kraftschlüssig mit dem Kunststoffelement verbindet. Dieser Form- und/oder Kraftschluss ist vorzugsweise so, dass er das brennbare Material beispielweise beim Transport und/oder bei Benutzung der Kerze im Verbund mit dem Kunststoffelement hält, dass dieser Verbund aber mit menschlichen Kräften vergleichsweise leicht überwunden werden kann, so dass die Kerze aus der Mulde entfernt werden kann, sofern dies erwünscht ist.

Gemäß einer bevorzugten Ausführungsform weist das Kunststoffelement eine Verbindungsfläche auf, an der eine Deckelfolie befestigt, insbesondere gesiegelt, werden kann. Durch diese bevorzugte Ausführungsform der vorliegenden Erfindung ist jedes Kunststoffelement abgeschlossen und dadurch das darin befindliche brennbare Material bzw. der Docht geschützt. Insbesondere bei sogenannten Duftkerzen wird durch eine Deckelfolie ein Ausgasen des Duftstoffes vor dem Abbrennen verhindert. Des Weiteren kann diese Deckelfolie mit Informationen oder Warenzeichen oder dergleichen bedruckt werden. Die Deckelfolie kann mit jedem beliebigen dem Fachmann geläufigen Verfahren bedruckt werden. Die Verbindungsfläche ist vorzugsweise der beschriebene umlaufende Flansch, an den die Deckelfolie gesiegelt wird. Insbesondere erfolgt das Siegeln durch Ultraschall. Gemäß einer bevorzugten Ausführungsform weist die Deckelfolie zumindest teilweise Gelatine und/oder Polysacharid und/oder Cellulose auf. Ganz besonders bevorzugt ist die Deckelfolie aus demselben Material hergestellt wie das Kunststoffelement. Noch mehr bevorzugt ist die Deckelfolie aus einem siegel- und/oder schweißfähigen Material hergestellt. Hierdurch kann eine abgedichtetes Kunststoffelement erhalten werden, welches mit einer flüssigen oder gelartigen Brennmasse gefüllt werden kann. Derartige Brennmassen können einen oder mehrere Duft- und/oder Farbstoffe aufweisen und sind deutlich kostengünstiger herzustellen als feste Brennmassen.

Das Kunststoffelement ist als becherartiges Gefäß ausgebildet, wobei das Kunststoffelement einen Bodenbereich und eine dem Bodenbereich gegenüberliegende Öffnung aufweist, wobei die Kerze einen Deckel aufweist, der die Öffnung verschließt. Bevorzugt verschließt der Deckel das Kunststoffelement flüssigkeitsdicht, so dass in dem Kunststoffelement ein brennbares Material vorgesehen werden kann, welches flüssig ist. Der Deckel kann ebenfalls aus einem Biopolymer, insbesondere demselben Biopolymer wie das Kunststoffelement, gebildet sein. Der Deckel kann als Platte, als Folie oder als becherartiges Gefäß ausgestaltet sein. Bevorzugt weist der Deckel eine Durchführung zum Durchführen des Dochts auf. Besonders bevorzugt ist an dem Deckel im Bereich der Durchführung ein Dochthalter zum Halten des Dochts angeordnet, der bevorzugt aus demselben Biopolymer wie der Deckel gebildet ist. Der Dochthalter ist bevorzugt einstückig mit dem Deckel ausgebildet.

Alternativ oder zusätzlich kann die erfindungsgemäße Kerze insgesamt in einer Verpackung, beispielsweise einer Schlauchbeutelverpackung, vorgesehen werden. Diese Verpackung kann ebenfalls mit Warenzeichen, Produktinformationen oder dergleichen bedruckt werden. Vorzugsweise ist diese Verpackung vollständig kompostierbar. Besonders bevorzugt ist die Verpackung aus demselben Material wie das Kunststoffelement.

Gemäß einer bevorzugten Ausführungsform weist die Kerze ein Einlegemittel auf. Insbesondere ein farbiges und/oder bedrucktes Einlegemittel. Besonders bevorzugt ist das Einlegemittel vereinzelt oder vereinzelbar im Bereich des Kunststoffelements angeordnet, insbesondere im Bereich der dem Bodenbereich gegenüberliegenden Öffnung. Hierdurch ist es in vorteilhafter Weise möglich, Informationen, Warnungen und/oder Werbehinweise in den einzelnen Kerzen vorzusehen. Ferner brennen die Kerzen mit dem Einlegemittel gleichmäßiger ab. Im Falle von Werbehinweisen sind diese vorteilhafterweise gut von oben über die gesamte Brenndauer der Kerze hinweg ablesbar.

Gemäß einer bevorzugten Ausführungsform ist das Einlegemittel rund oder eckig vorgesehen, insbesondere entspricht die äußere Kontur des Einlegemittels einem inneren Querschnitt des Kunststoffelements. Besonders bevorzugt ist das Einlegemittel im Wesentlichen scheiben- oder plättchenförmig ausgebildet. Ganz besonders bevorzugt ist das Einlegemittel auf dem brennbaren Material angeordnet. Noch mehr bevorzugt ist das Einlegemittel form-, kraft- und/oder stoffschlüssig mit dem brennbaren Material verbunden, insbesondere auf dieses aufgeklebt, vorzugsweise mit einem nicht brennbaren Klebstoff. Hierdurch wird vorteilhafterweise sichergestellt, dass das Einlegemittel beim Transport bzw. Gebrauch der Kerze nicht verrutscht. Vorzugsweise ist das Einlegemittel aus einem Material gefertigt, das zumindest teilweise Gelatine und/oder Polysacharid und/oder Cellulose aufweist. Besonders bevorzugt ist es dasselbe Material, das auch zum Herstellen des Kunststoffelements eingesetzt wird.

Vorzugsweise weist das Einlegemittel eine, insbesondere mittig angeordnete, Öffnung auf, in der der Docht angeordnet werden kann bzw. durch die der Docht durchführbar ist.

Gemäß einer bevorzugten Ausführungsform weist das Kunststoffelement im oberen, dem Boden gegenüberliegenden Bereich einen umlaufenden Rand auf. Besonders bevorzugt ist der Rand im Wesentlichen horizontal vorgesehen, insbesondere in einem Winkel zu der Seitenwand von ungefähr 90 Grad, wobei ganz besonders bevorzugt zwischen dem Rand und der Seitenwand ein Krümmungsradius vorgesehen ist. Alternativ ist der Rand gegenüber der Horizontalen geneigt vorgesehen. Ganz besonders bevorzugt ist der Rand als Verbindungsfläche vorgesehen. Noch mehr bevorzugt weist der Rand eine umlaufende Prägung, insbesondere eine Erhebung wie z.B. einen Grat, auf. Durch die Vorsehung eines Randes wird sichergestellt, dass die Kerze besser von einem Benutzer gefasst werden kann, insbesondere auch in einem brennenden bzw. heißen Zustand. Zudem wird vorteilhafterweise die Stabilität des Kunststoffelements durch einen solchen Rand deutlich erhöht. Dabei ist ein horizontaler Rand, bzw. ein Rand, der mit der Seitenwand einen rechten Winkel einschließt, stabiler als ein geneigter Rand. Ein geneigter Rand, bzw. eine Prägung bietet den Vorteil einer besseren Siegelfläche für die Verbindung mit der Deckelfolie. Insbesondere eine Prägung ermöglicht ein schnelles und einfaches Ansiegeln einer Deckelfolie, ohne dass die Siegelvorrichtung, beispielsweise eine Sonotrode bei einer Ultraschallsiegelung, exakt zu dem Rand ausgerichtet werden muss.

Vorzugsweise weist das Kunststoffelement einen Bodenbereich und eine Seitenwand sowie eine dem Bodenbereich gegenüberliegende Öffnung auf. Besonders bevorzugt weist die Mulde eine rechteckige, insbesondere quadratische, runde, sternförmige, oder polygonale horizontale Querschnittsfläche auf. Ganz besonders bevorzugt ist die Seitenwand gegenüber der Vertikalen geneigt vorgesehen und/oder ist der Bodenbereich gegenüber der Horizontalen geneigt vorgesehen, insbesondere so geneigt, dass das brennbare Material im flüssigen Zustand entlang des Bodens in Richtung des Dochts, der sich in der Mitte des Bodens befindet, fließt. Hierdurch wird eine flexible Verwendung in Behältnissen unterschiedlichen Durchmessers ermöglicht, zudem kann das brennbare Material entlang der Neigung besser in Richtung des Dochtes fließen und somit ein verbesserter und möglichst vollständiger Abbrand der Kerze sichergestellt werden.

Der Fachmann versteht, dass sich Begriffe wie horizontal oder vertikal auf eine übliche Ausrichtung einer Mulde bzw. Kerzen beziehen, also insbesondere auf eine Positionierung der Mulde bzw. Kerze auf einer ebenen Fläche. Der Fachmann erkennt weiterhin, dass die Ausrichtung der Kerze nicht auf eine solche Ausrichtung beschränkt ist.

Gemäß einer bevorzugten Ausführungsform ist der Docht als Endlosband vorgesehen, wobei besonders bevorzugt an dem Endlosband, insbesondere in regelmäßigen Abständen, Dochthalter vorgesehen, insbesondere angeformt, sind.

Gemäß einer bevorzugten Ausführungsform weist da Kunststoffelement ein Stabilisierungsmittel auf. Besonders bevorzugt ist das Stabilisierungsmittel wenigstens eine Prägung der Seitenwand, insbesondere eine um den ganzen Umfang der Seitenwand umlaufende Ein- und/oder Ausbuchtung. Alternativ oder zusätzlich ist das Stabilisierungsmittel wenigstens eine Prägung in einem Übergangsbereich zwischen dem Bodenbereich und der Seitenwand. Vorzugsweise ist das Stabilisierungsmittel als eine Sicke oder Doppelsicke, insbesondere in dem o.g. Bereich, vorgesehen. Hierdurch ist es in vorteilhafter Weise möglich, dass eine thermische Rückformung bzw. Rückstellung des Materials, aus dem die Mulde gefertigt ist, zumindest vermindert wird, beispielsweise wenn der Übergangsbereich beim Abbrand der Kerze erwärmt wird.

Gemäß einer bevorzugten Ausführungsform weist das Kunststoffelement auf seiner Unterseite, wenigstens eine Auflagefläche, insbesondere einen Fuß, auf, auf dem die Kerze auf einer Unterlage aufliegt. Besonders bevorzugt ist die Auflagefläche einstückig mit dem Bodenbereich hergestellt, d.h. ebenfalls durch Tiefziehen und/oder Spitzen gefertigt. Ganz besonders bevorzugt ist der Fuß eine wenigstens abschnittsweise umlaufende, konzentrische Prägung oder Materialanhäufung. Alternativ oder zusätzlich ist der Fuß eine lokale, insbesondere halbkugelförmige oder quaderförmige, Prägung/Materialanhäufung. Hierdurch wird eine Auflagefläche zur Verfügung gestellt, die vorteilhafterweise einen stabilen Stand der Kerze ermöglicht, was die Betriebssicherheit der Kerze erhöht. Zudem wird eine Luftzirkulation auf der Unterseite des Kunststoffelements ermöglicht, wodurch ein Wärmestau vermieden wird und der Abbrand der Kerze kontrollierter und langsamer verläuft. Auch wird eine Beeinträchtigung der Fläche, auf der die Kerze abgestellt wird, vermieden.

Gemäß einer bevorzugten Ausgestaltung weist das Kunststoffelement ein Halteelement auf. Das Halteelement kann einen Sockel des Kunststoffelements ausbilden. Alternativ kann das Halteelement ein Verbindungselement zum Verbinden des Kunststoffelements mit einer Vorrichtung zum Halten einer Kerze sein. Das Verbindungselement ist bevorzugt derart ausgebildet, dass es die Vorrichtung zum Halten der Kerze in einer Verbindungsstellung umgreift. Bei der Vorrichtung zum Halten der Kerze kann es sich beispielsweise um einen Teil eines Opferkerzentischs handeln.

Das brennbare Material wird vorzugsweise als flüssiges oder pastöses Medium in das Kunststoffelement eingefüllt und härtet dort aus. Wird das Element auslaufsicher versiegelt, kann die Brennmasse flüssig sein.

Vorzugsweise werden die Kunststoffelemente vor, beim und/oder nach dem Einfüllen des brennbaren Materials gekühlt, um das Härten des brennbaren Materials zu beschleunigen.

Beschrieben wird ferner ein Docht, wobei der Docht in einem Brennstoppbereich ein Biopolymer, insbesondere Gelatine und/oder Polysacharid und/oder Cellulose, besonders bevorzugt Gelatine und/oder Hydroxypropylmethylcellulose und/oder Pullulan aufweist. Über den Brennstoppbereich kann ein Abbrennen des Dochts über den Brennstoppbereich hinaus verhindert werden. Insofern ist es möglich, einen Docht mit einer Selbstlöschfunktion bereitzustellen. Bevorzugt ist der Docht in dem Brennstoppbereich mit dem Biopolymer getränkt.

Der Docht kann bei einer Kerze und/oder einer Flüssigbrennstofflampe, beispielsweise einer Öllampe, Verwendung finden. Bevorzugt findet der Docht Anwendung bei einer Kerze mit einem Kunststoffelement, welches zumindest teilweise oder vollständig aus einem Biopolymer besteht.

Gemäß einer bevorzugten Ausgestaltung ist der Docht in dem Brennstoppbereich farbig markiert, so dass der Brennstoppbereich bei der Verarbeitung des Dochts vereinfacht erkannt werden kann.

Beschrieben wird ferner ein Streichholz wobei das Streichholz in einem Brennstoppbereich ein Biopolymer, insbesondere Gelatine und/oder Polysacharid und/oder Cellulose, besonders bevorzugt Gelatine und/oder Hydroxypropylmethylcellulose und/oder Pullulan aufweist. Über den Brennstoppbereich kann ein Abbrennen des Streichholzes über den Brennstoppbereich hinaus verhindert werden. Insofern ist es möglich, ein Streichholz mit einer Selbstlöschfunktion bereitzustellen. Bevorzugt ist das Streichholz in dem Brennstoppbereich mit dem Biopolymer getränkt.

Ferner beschrieben wird ein Duftkörper, welcher einen Duftstoff aufweist, wobei der Duftkörper ein Biopolymer, insbesondere Gelatine und/oder Polysacharid und/oder Cellulose, besonders bevorzugt Gelatine und/oder Hydroxypropylmethylcellulose und/oder Pullulan umfasst. Hierdurch kann ein unerwünschtes Brennen des Duftkörpers verhindert werden.

Der der beschriebene Duftkörper kann beispielsweise erwärmt werden, um den Duftstoff abzugeben. Bei einem Erwärmen des Duftkörpers mit offenem Feuer, beispielsweise mit einer Kerze, kann ein unerwünschtes Brennen des Duftkörpers verhindert werden.

Im Folgenden wird die Erfindung anhand von den Figuren 1-21 näher erläutert:
- **Figur 1**: zeigt eine Kerze.
- **Figuren 2 und 3**: zeigen eine Kerze.
- **Figur 4**: zeigt noch eine Kerze
- **Figuren 5 und 6**: zeigen ein Herstellungsverfahren für eine Kerze (nicht erfindungsgemäss)
- **Figur 7**: zeigt eine Kerze mit einem Auflageplättchen
- **Figur 8**: zeigt den Dochthalter
- **Figuren 9 und**: **10** zeigen beispielhafte Kunststoffelemente
- **Figur 11**: zeigt eine Einbuchtung für den Docht oder den Dochthalter.
- **Figur 12**: zeigt eine weitere Kerze
- **Figur 13**: zeigt eine weitere Kerze
- **Figur 14**: zeigt eine weitere Kerze
- **Figur 15a**: zeigt ein Ausführungsbeispiel einer Form zur Herstellung der Kerze
- **Figur 15b**: zeigt ein Ausführungsbeispiel einer mit der Form nach Figur 15a hergestellten Kerze
- **Figur 16a**: zeigt ein Ausführungsbeispiel einer Form zur Herstellung der Kerze
- **Figur 16b**: zeigt ein Ausführungsbeispiel einer mit der Form nach Figur 16a hergestellten Kerze
- **Figur 17**: zeigt eine Vorrichtung zur Durchführung eines Herstellungsverfahrens für einen Docht und eine Kerze
- **Figur 18**: zeigt einen Streichholz
- **Figur 19**: zeigt einen Duftkörper
- **Figur 20**: zeigt eine Kerze
- **Figur 21**: zeigt eine Kerze

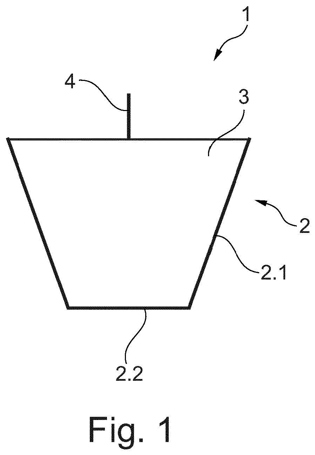

**Figur 1** zeigt die Kerze 1, die aus einem brennbaren Material 3, beispielsweise einem Wachs, wachsähnlichen Material oder technischem Wachsgemenge besteht, in dem ein Docht 4 vorgesehen ist. Das brennbare Material 3 und der Docht 4 sind in einem Kunststoffelement 2 vorgesehen. Dieses Kunststoffelement 2, das hier einen Boden- 2.2 und einen Wandbereich aufweist, besteht zumindest teilweise aus Gelatine und/oder Polysacharid und/oder Cellulose, besonders bevorzugt Gelatine und/oder Hydroxypropylmethylcellulose und/oder Pullulan, insbesondere HPMC. Diese Gelatine und/oder Polysacharid und/oder Cellulose kann vernetzt sein und ist vorzugsweise mit mindestens einer Substanz versetzt, die die Brennbarkeit der Gelatine und/oder des Polysacharids und/oder der Cellulose reduziert und/oder deren Glastemperatur erhöht. In dem vorliegenden Fall ist die Kerze ein sogenanntes Teelicht, bei dem das Kunststoffelement 2 das brennbare Material 3 und den Docht 4 vollständig aufnimmt. Das brennbare Material wird mittels des Dochts in dem Kunststoffelement 2 abgebrannt. Danach wird das Kunststoffelement 2 entsorgt, indem es beispielsweise kompostiert wird. Das Kunststoffelement kann aus einer Folie tiefgezogen oder mit einem flüssigen oder viskosen Material gespritzt und/oder gegossen werden. In jedem Fall weist das Material, aus dem das Kunststoffelement hergestellt wird zumindest teilweise Gelatine und/oder Polysacharid und/oder Cellulose, besonders bevorzugt Gelatine und/oder Hydroxypropylmethylcellulose und/oder Pullulan insbesondere HPMC auf.

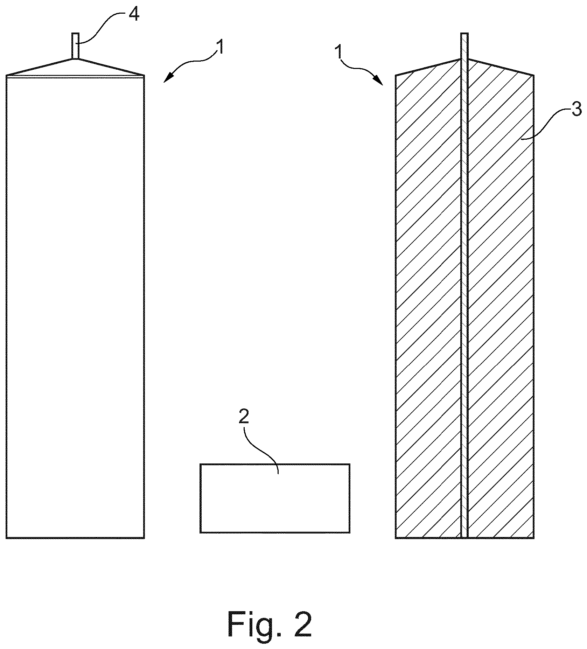

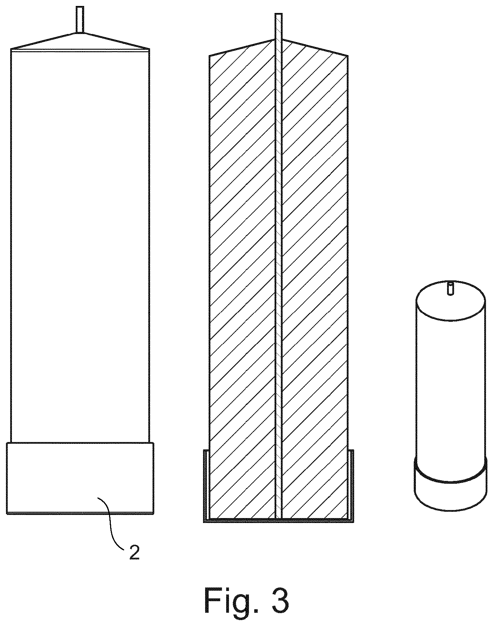

**Figuren 2** **und** **3** zeigen eine Kerze. Diese besteht aus einem Kunststoffelement 2, einen Docht 4 und ein brennbares Material 3, das den Docht umgibt. Das Kunststoffelement 2 weist einen Bodenbereich und einen hier zylindrischen Wandbereich auf. Das brennbare Material steht auf dem Bodenbereich auf. Die Höhe des Wandbereichs ist in dem vorliegenden Fall kleiner als die Höhe des brennbaren Materials. Gemäß einer anderen Ausführungsform entspricht die Höhe des brennbaren Materials der Höhe der Seitenwand oder die Höhe der Seitenwand übersteigt sogar die Höhe des brennbaren Materials. Die Innenkontur des Kunststoffelementes kann so vorgesehen sein, dass das Kunststoffelement die Außenkontur des brennbaren Elementes im Wesentlichen spaltfrei umschließt. Es ist aber auch möglich, dass die Kontur der Seitenwand kleiner oder größer als die Kontur des brennbaren Materials vorgesehen ist, so dass die Seitenwand des Kunststoffelements nicht zu sehen ist oder ein Spalt zwischen der Innenkontur der Seitenwand des Kunststoffelementes vorgesehen ist. Das Kunststoffelement kann demnach auch als Kerzenhalter, beispielsweise bei einem Adventskalender dienen. Es verhindert dann das Auslaufen von Wachs und dient als Brennstopp für den Docht.

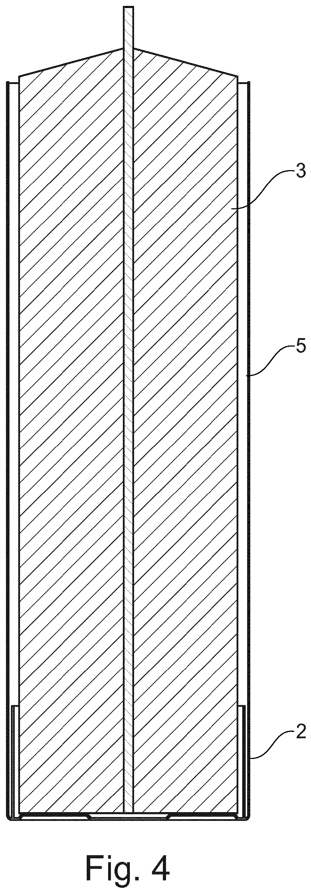

**Figur 4** zeigt noch eine Kerze. Im Wesentlichen kann auf die Ausführungen zu den voranstehenden Figuren Bezug genommen werden, wobei in dem vorliegenden Fall der Teil des brennbaren Materials, der nicht von dem Kunststoffelement 2 umgeben ist, mit einem Überzug 5 versehen ist. Vorzugsweise ist dieser Überzug 5 farbig vorgesehen. Vorzugsweise sind der Außendurchmesser des Überzugs 5 und der Außendurchmesser des Kunststoffelementes bündig vorgesehen. Der Überzug 5 ist vorzugsweise aus einem brennbaren Material hergestellt.

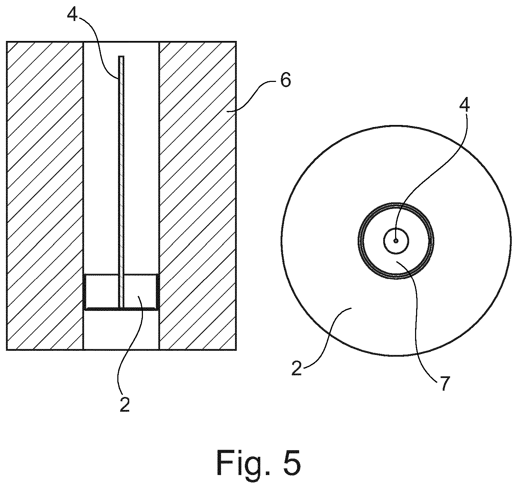

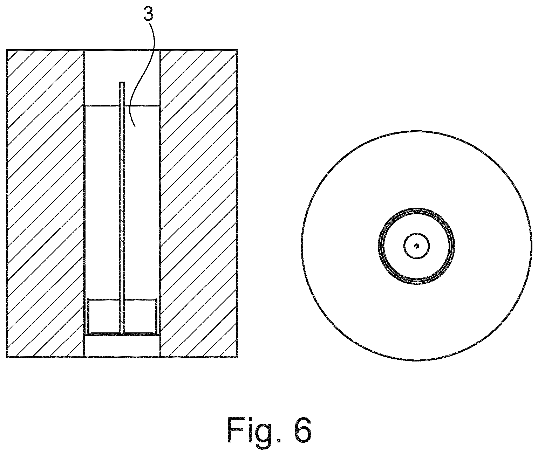

**Figuren 5** **und** **6** zeigen ein Herstellungsverfahren für eine Kerze. Das Kunststoffelement 2 wird in einer Gießform 6 vorgesehen. Der Docht 4 befindet sich ebenfalls in dem Hohlraum der Gießform 6 und ist vorzugsweise mit dem Kunststoffelement verbunden, beispielsweise indem der Docht einen Dochthalter 7 aufweist, der mit dem Kunststoffelement 2 verbunden ist. Sobald das Kunststoffelement und der Docht in dem Hohlraum der Gießform 6 platziert worden sind, wird dieser zumindest teilweise mit dem flüssigen brennbaren Material gefüllt. Sobald dieses erstarrt ist, kann die so hergestellte Kerze aus der Gießform entformt werden.

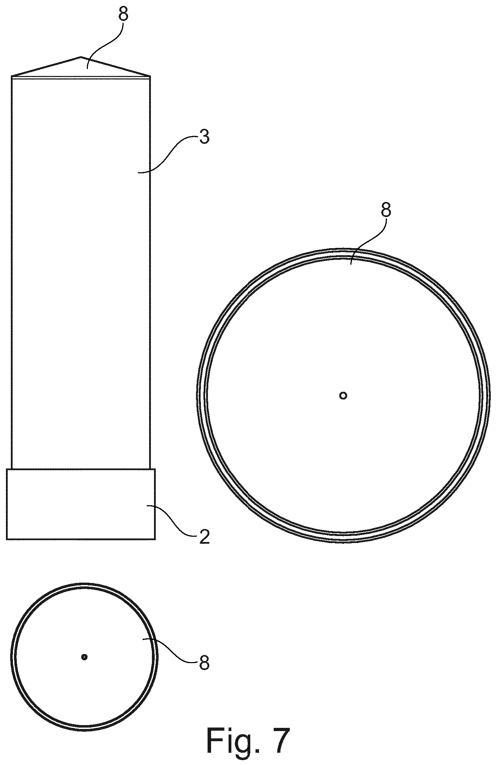

**Figur 7** zeigt eine Kerze mit einem Auflageplättchen 8, das mit einem "Text" und/oder einem Bild bedruckt sein kann und eine Ausnehmung für den Docht aufweist. Vorzugsweise ist diese Auflageplättchen aus demselben Material wie das Kunststoffelement gefertigt und/oder weist zumindest Gelatine und/oder Polysacharid auf.

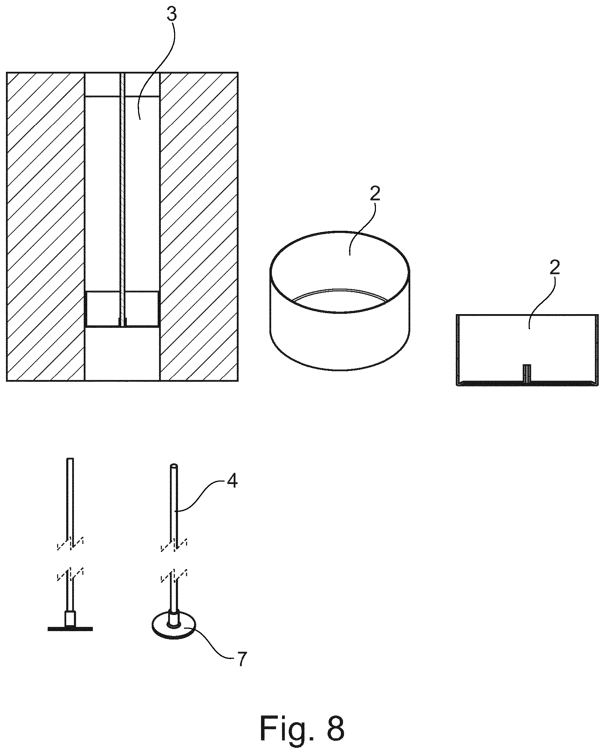

**Figur 8** zeigt einen Dochthalter 7, der mit dem Docht verbunden ist. Vorzugsweise ist der Dochthalter auch mit dem Kunststoffelement 2 verbunden, beispielsweise verklebt. Vorzugsweise basiert der Kleber auch auf Gelatine und/oder Polysacharid. Vorzugsweise ist diese Dochthalter aus demselben Material wie das Kunststoffelement gefertigt und/oder weist zumindest Gelatine und/oder Polysacharid auf, besonders bevorzugt Gelatine und/oder Hydroxypropylmethylcellulose und/oder Pullulan.

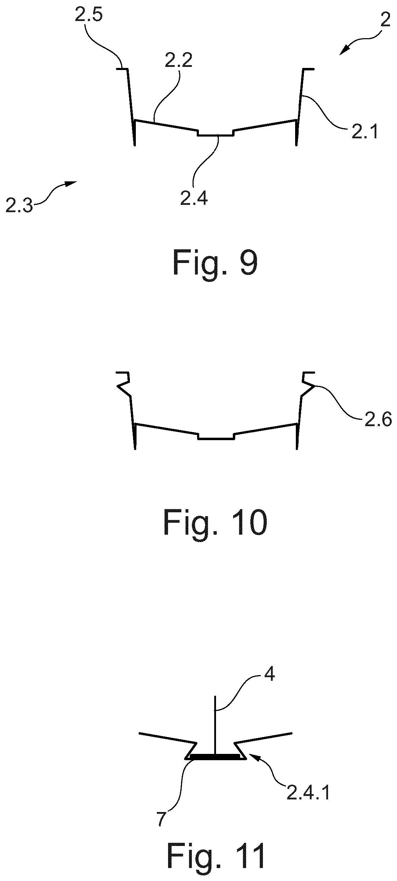

**Figur 9** zeigt Kunststoffelement 2. Dieses weist eine Seitenwand 2.1, vorzugsweise eine gegenüber der Vertikalen geneigte Seitenwand, auf, wobei der Querschnitt des Kunststoffelements 2 zu einem Bodenbereich 2.2 hin vorzugsweise abnimmt. Des Weiteren weist das Kunststoffelement 2 einen Bodenbereich 2.2 auf, der in dem vorliegenden Fall gegenüber der Horizontalen in Richtung der Mitte des Kunststoffelements 2 geneigt ist, so dass das in dem Kunststoffelement 2 aufgenommene verflüssigte, brennbare Material 3 in Richtung des Dochtes 4 fließt. Vorzugsweise ist in dem Bodenbereich 2.3 eine Ein- oder Ausbuchtung, hier eine Einbuchtung 2.4, vorgesehen, in der der Docht 4 und/oder ein Dochthalter 7 angeordnet werden kann. Der Docht 4 und/oder der Dochthalter 7 sind vorzugsweise form-, kraft- und/oder stoffschlüssig mit dem Bodenbereich 2.2, insbesondere der Einbuchtung 2.4 verbunden. Mit der Ein- und/oder Ausbuchtung 2.4 wird verhindert, dass sich der Docht 4 beim Einfüllen des brennbaren Materials 3 und/oder beim Abbrand der Kerze 1 aus seiner zentrierten Lage entfernt. Weiterhin bevorzugt sind der Bodenbereich 2.2 und/oder die Seitenwände 2.1 so vorgesehen, dass der Bodenbereich 2.2, in dem sich der Docht 4 und/oder der Dochthalter 7 befindet, beabstandet von einer Auflagefläche 2.3 vorgesehen ist. Dadurch wird sichergestellt, dass die Auflagefläche 2.3, auf der die Kerze 1 steht, beim Abbrand der Kerze 1 nicht beschädigt wird. In dem vorliegenden Fall wird dies dadurch erreicht, dass die Seitenwände 2.1 tiefer gezogen sind als der Bodenbereich 2.2, 2.4. Alternativ oder zusätzlich kann der Bodenbereich 2.2, 2.4 jedoch auch Auswölbungen aufweisen, mit denen das Kunststoffelement 2 auf der Auflagefläche 2.4 steht und die einen Abstand zwischen dem Bodenbereich 2.3, 2.4, in dem sich der Docht 4 oder der Dochthalter 7 befindet, und der Auflagefläche 2.3 bewirken. Gegenüberliegend von dem Bodenbereich 2.3 weist die Seitenwand 2.1 in dem vorliegenden Fall einen Rand 2.5, hier einen umlaufenden Rand, auf, der als Siegelfläche für einen möglicherweise vorhandenen Deckel 16 insbesondere eine Deckelfolie, dienen kann. Darüber hinaus gibt dieser Rand 2.5 dem Kunststoffelement 2 jedoch auch eine zusätzliche Stabilität. Vorzugsweise kann insbesondere die Seitenwand 2.1 zusätzliche Ein- und/oder Ausbuchtungen aufweisen, insbesondere um deren Stabilität zu erhöhen.

**Figur 10** zeigt Kunststoffelement 2. Im Wesentlichen kann auf die zu **Figur 9** gemachten Ausführungen Bezug genommen werden. In dem vorliegenden Fall ist zusätzlich in der Seitenwand 2.1 ein Form- und/oder Kraftschlussmittel 2.6, hier ein Hinterschnitt, vorgesehen, der form- und/oder kraftschlüssig mit dem brennbaren Material 3 der Kerze 1 zusammenwirkt. Durch diesen Form- und/oder Kraftschluss wird verhindert, dass sich das brennbare Material 3 und der Docht 4, d.h. die Kerze 1, ungewollterweise von dem Kunststoffelement 2 lösen. Der Form- und/oder Kraftschluss wird insbesondere dadurch erzielt, dass flüssiges brennbares Material 3 in das Kunststoffelement 2 eingeführt wird und dort erstarrt. Beim Erstarren entsteht der Form- und/oder Kraftschluss. Dieses Form- und/oder Kraftschlussmittel 2.6 ist insbesondere dann bevorzugt, wenn die Kerze 1 für den Abbrand aus dem Kunststoffelement 2 entfernt werden soll. Dafür wird beispielsweise ein Druck auf den Bodenbereich ausgeübt, der die Kerze 1 aus dem Kunststoffelement 2 herausdrückt. Dabei löst sich der Form- und/oder Kraftschluss zwischen dem brennbaren Material 3 und dem Kunststoffelement 2, vorzugsweise schnappend, und die Kerze 1 kann ohne das Kunststoffelement 2, beispielsweise als Schwimmkerze, eingesetzt werden.

**Figur 11** zeigt Details der Ein- und/oder Ausbuchtung 2.4 für den Docht 4. Es ist deutlich zu erkennen, dass die Seitenwände 2.4.1 der Einbuchtung mit einem Hinterschnitt versehen sind, so dass sich eine form- und/oder kraftschlüssige Verbindung zwischen dem Docht 4 bzw. dem Dochthalter 7 und dem Bodenbereich des Kunststoffelements 2 ergibt. Diese oder jede anders geformte Einbuchtung kann beispielweise dadurch hergestellt werden, dass der Dochthalter 7 beim Tiefziehen der Mulde 2 beispielsweise mit einem Stempel in die Kunststofffolie hineingedrückt wird und sich dabei die gewünschte Tiefziehform ergibt. Beim Entfernen des Stempels verbleibt der Docht 4 und/oder der Dochthalter 7 dann in dem so fertiggestellten Kunststoffelement 2 und ist vorzugsweise form- und/oder kraftschlüssig mit diesem verbunden.

Der Fachmann versteht, dass auf diese Weise auch ein Stoffschluss, beispielsweise durch (Ultraschall-)Siegeln oder Kleben mit einem Klebstoff, insbesondere einem Schmelzklebstoff, einem sogenannten Hotmeltkleber, zwischen dem Docht 4 bzw. dem Dochthalter 7 und dem Bodenbereich 2.2, 2.4 des Kunststoffelements 2 erzielt werden kann.

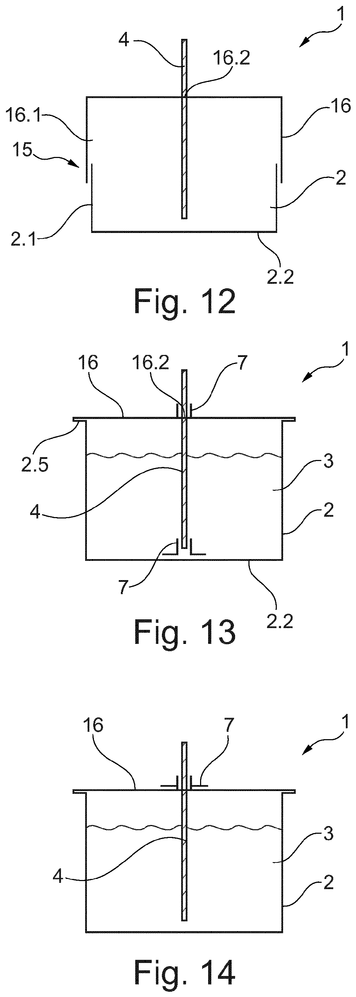

**Figur 12** zeigt eine Kerze 1. Hier ist das Kunststoffelement 2 als Mulde ausgebildet, in welcher das brennbare Material aufgenommen werden kann. Das Kunststoffelement 2 weist bevorzugt eine kreisförmige Grundfläche auf. Das Kunststoffelement 2 weist einen Bodenbereich 2.2 und eine mit dem Bodenbereich verbundene Seitenwand 2.1 auf. Auf der dem Bodenbereich 2.2 abgewandten Seite der Seitenwand 2.1 ist eine Öffnung vorgesehen. Die Öffnung des Kunststoffelements 2 ist mittels einem Deckel 16 verschlossen. Der Deckel 16 ist aus demselben Material wie das Kunststoffelement 2 gebildet. Das bedeutet, dass sowohl das Kunststoffelement 2 als auch der Deckel 16 ein Biopolymer enthalten. In einem Verbindungsbereich 15 sind das Kunststoffelement 2 und der Deckel 16 miteinander verbunden, insbesondere verklebt, verklemmt oder versiegelt. Hierzu ist der Deckel 16 derart bemessen, dass er über das Kunststoffelement 2 gestülpt werden kann. Beispielsweise kann der Deckel 16 Seitenwände 16.1 aufweisen, die zur Verbindung mit dem Kunststoffelement 2 in Anlage mit dem Kunststoffelement gebracht werden. Insofern wird ein geschlossener Behälter zur Aufnahme des brennbaren Materials bereitgestellt. Wenn die Verbindung zwischen dem Kunststoffelement 2 und dem Deckel 16 flüssigkeitsdicht ausgebildet ist, kann in dem geschlossenen Behälter ein flüssiges, brennbares Material aufgenommen werden, ohne das ein unerwünschtes Auslaufen zu befürchten ist. In dem Deckel ist eine Durchführung 16.2 vorgesehen, durch welche der Docht 4 der Kerze 1 geführt ist. Über den Docht 4 kann brennbares Material aus dem Inneren des durch das Kunststoffelement 2 und den Deckel 16 gebildeten geschlossenen Behälters nach außen verbracht werden, um dort zu verbrennen.

In **Figur 13** ist eine Kerze 1. Hier ist das Kunststoffelement 2 als Mulde ausgebildet, in welcher das brennbare Material aufgenommen werden kann. Das Kunststoffelement 2 weist bevorzugt eine kreisförmige Grundfläche auf. Das Kunststoffelement 2 weist einen Bodenbereich 2.2 und eine mit dem Bodenbereich verbundene Seitenwand 2.1 auf. Auf der dem Bodenbereich 2.2 abgewandten Seite der Seitenwand 2.1 ist eine Öffnung vorgesehen. Die Öffnung wird durch einen Rand 2.5 des Kunststoffelements umrandet, welcher das Kunststoffelement versteift und gleichzeitig einen Verbindungsbereich zur Verbindung mit einem Deckel 16 bereitstellt. Die Öffnung des Kunststoffelements 2 ist mit einem Deckel 16 verschlossen. Der Deckel 16 ist aus demselben Material wie das Kunststoffelement 2 gebildet. Bei dem Deckel 16 handelt es sich um einen als Platte oder als Folie ausgebildeten Deckel 16. Der Deckel ist flüssigkeitsdicht mit dem Kunststoffelement 2 verbunden, beispielsweise versiegelt oder verklebt. In dem durch das Kunststoffelement 2 und den Deckel 16 gebildeten geschlossenen Behälter ist ein, insbesondere flüssiges, brennbares Material 3 aufgenommen. In dem Deckel 16 ist eine Durchführung 16.2 vorgesehen, durch welche der Docht 4 der Kerze 1 geführt ist. Über den Docht 4 kann brennbares Material aus dem Inneren des durch das Kunststoffelement 2 und den Deckel 16 gebildeten geschlossenen Behälters nach außen verbracht werden, um dort zu verbrennen. Zur Befestigung des Dochts 4 ist am Bodenbereich 2.2 des Kunststoffelements 2 und/oder an dem Deckel 16 im Bereich der Durchführung 16.2 ein Dochthalter 7 angeordnet. Der oder Die Dochthalter 7 sind aus demselben Material wie das Kunststoffelement 2 ausgebildet. Insofern enthalten der oder die Dochthalter 7 ein Biopolymer.

Die **Figur 14** zeigt eine Kerze 1.

Dieses Ausführungsbeispiel gleicht dem in Figur 13 gezeigten Ausführungsbeispiel, so dass die diesbezügliche Beschreibung auch auf dieses Ausführungsbeispiel zutrifft. Im Unterschied zu dem Ausführungsbeispiel nach Figur 13 ist lediglich an dem Deckel 16 ein Dochthalter 7 angeordnet. Der Dochthalter 7 ist aus demselben Material wie der Deckel 16 ausgebildet und bevorzugt einstückig mit dem Deckel 16 vorgesehen.

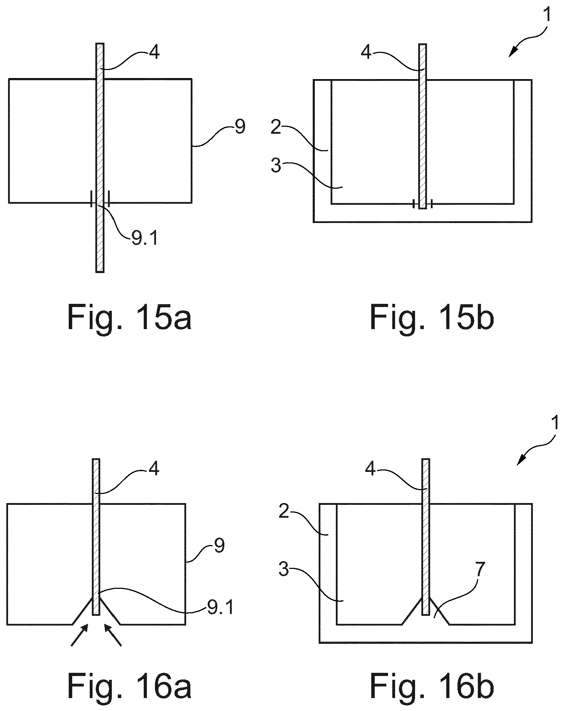

**Figur 15a** zeigt ein Ausführungsbeispiel einer Form 9 zur Herstellung der Kerze 1. Die Form 9 ist als Tauchform ausgebildet und weist eine Außenkontur mit einem Tauchbereich auf, welcher in das flüssige Biopolymer eingetaucht wird. Der Tauchbereich entspricht einem Negativ der Innenkontur des herzustellenden Kunststoffelements 2. Die Form 9 weist an einer Unterseite eine Durchführung 9.1 für den Docht 4 auf. Der Docht 4 verläuft durch diese Durchführung 9.1. Der Docht 4 kann zusammen mit der Form 9 in das flüssige Biopolymer eingetaucht werden, so dass ein Kunststoffelement mit einem Bodenbereich gebildet wird, welcher mit dem Docht 4 verbunden ist. Ein separater Dochthalter ist in diesem Fall nicht erforderlich. Die Form 9 kann dann zusammen mit dem Docht 4 aus dem flüssigen Biopolymer herausgezogen werden. Das Biopolymer verfestigt sich und bildet ein mit dem Docht 4 verbundenes Kunststoffelement 2 nach Art einer Mulde. Der Docht 4 kann dann in Höhe des Bodenbereichs des Kunststoffelements verschlossen werden. Die verbleibende Öffnung unterhalb des Dochts 4 wird verschlossen, beispielsweise dadurch, dass die Form 9 nochmals in ein flüssiges Biopolymer eingetaucht wird, so dass sich an der Außenkontur des gebildeten Kunststoffelements 2 eine weitere Lage des Biopolymers abscheidet, welche die Öffnung unterhalb des Dochts 4 verschließt, vgl. **Figur 15b****.**

Die **Figur 16a** zeigt ein weiteres Ausführungsbeispiel einer Form 9 zur Herstellung eines Kunststoffelements 2 für eine Kerze 1 gemäß der Erfindung. Diese Form 9 ist als Tauchform ausgebildet und weist eine Außenkontur mit einem Tauchbereich auf, welcher in das flüssige Biopolymer eingetaucht wird. Der Tauchbereich entspricht einem Negativ der Innenkontur des herzustellenden Kunststoffelements. Die Form 9 ist an ihrer Unterseite derart ausgestaltet, dass in dem erzeugten Kunststoffelement ein Dochthalter 7 zum Halten und/oder Einklemmen des Dochts 4 gebildet wird. Hierzu weist die Form 9 an ihrer Unterseite eine Durchführung 9.1 für den Docht 4 auf. Der Docht 4 verläuft durch diese Durchführung 9.1. Unterhalb der Durchführung ist Aussparung vorgesehen, in welche das Biopolymer zu Bildung des in das Kunststoffelement 2 integrierten Dochthalters einlaufen kann. Die Richtung des in die Aussparung einlaufenden Biopolymers ist Figur 16a durch Pfeile angedeutet. Die Form 9 kann aus dem flüssigen Biopolymer herausgezogen werden. Das Biopolymer verfestigt sich und bildet ein Kunststoffelement 2 nach Art einer Mulde mit integriertem Dochthalter 7. Der Docht 4 ist mit dem Dochthalter 7 verbunden, **vgl.** **Figur 16b****.** Gemäß einer Abwandlung kann die in Figur 16a dargestellte Form 9 ohne einen Docht 4 in das Biopolymer getaucht werden, so dass das in Figur 16b gezeigte Kunststoffelement 2 mit integriertem Dochthalter 7 aber ohne verbundenen Docht 4 gebildet wird. Der Docht 4 kann dann in einem weiteren Schritt durch Kleben und/oder Einklemmen mit dem Dochthalter 7 verbunden werden.

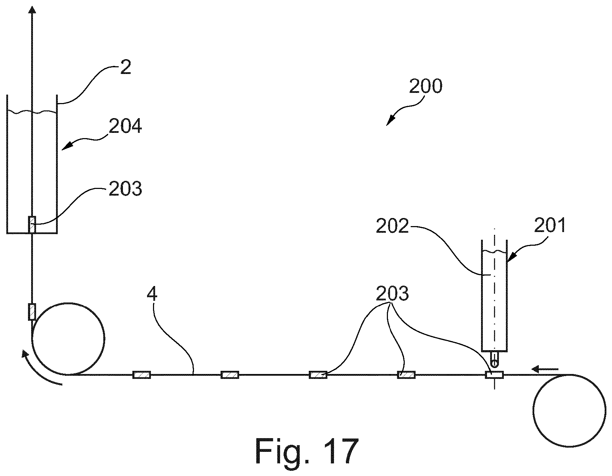

In **Figur 17** ist schematisch eine Vorrichtung 200 zur Durchführung eines Herstellungsverfahrens für einen Docht 4 und eine Kerze 1 gezeigt. Der brennbare Docht 4 wird zunächst einer Auftragsvorrichtung 201 zugeführt, in welcher der Docht 4 bereichsweise mit einem Biopolymer 202 getränkt wird oder bereichsweise ein Biopolymer 202 auf den Docht 4 aufgebracht wird. Hierbei werden entlang des Dochts 4 Brennstoppbereiche 203 gebildet. In dem Brennstoppbereich 203 ist der Docht 4 nicht brennbar und/oder entflammbar. Optional ist der Docht 4 in den Brennstoppbereichen 203 markiert, beispielsweise farblich markiert oder mit einem UV- oder IR-Marker versehen. In einem weiteren Schritt wird der Docht 4 einer Vorrichtung 204 zur Herstellung einer Kerze 1 zugeführt. In der Vorrichtung 204 wird der Docht 4 mit einem Kunststoffelement 2 zusammengeführt, welches ein Biopolymer aufweist. Die Zuführung des Dochts 4 in das Kunststoffelement 2 kann von oben - oder wie hier gezeigt - von unten erfolgen. Die Vorrichtung 204 zur Herstellung einer Kerze 1 weist einen Sensor auf, über welchen der Brennstoppbereich 203 erkannt werden kann. Beispielsweise kann dies ein optischer Sensor, insbesondere ein UV- oder IR-Sensor sein. Über den Sensor kann die Förderung des Dochts 4 derart gesteuert werden, dass ein Brennstoppbereich 203 zur Anlage mit einem Bodenbereich des Kunststoffelements 2 oder zur Anlage mit einem Dochthalter des Kunststoffelements kommt. Der Docht 4 kann dann mit dem Kunststoffelement und/oder dem Dochthalter verbunden werden. Schließlich wird der Docht 4 abgelängt.

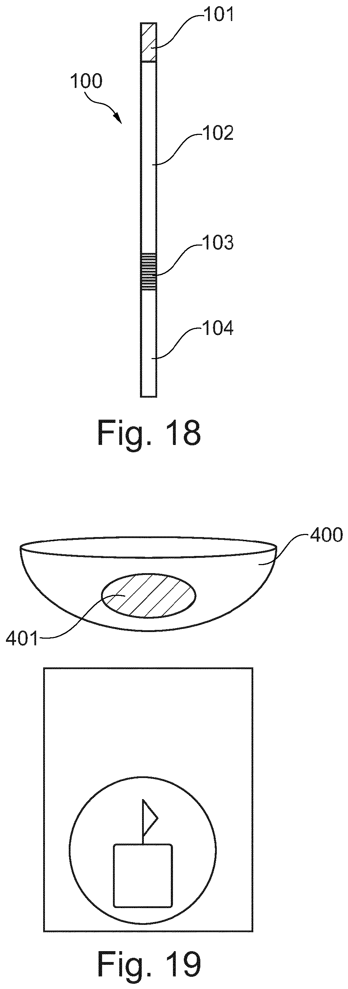

In der **Figur 18** ist ein Streichholz 1. Das Streichholz 100 weist einen Grundkörper auf, der beispielsweise aus Holz besteht. Das Streichholz weist einen Zündkopf 101 auf. Im Bereich des Zündkopfs ist das Streichholz 100 mit einem entflammbaren Material beschichtet. Ferner weist das Streichholz 100 einen Brennstoppbereich 103 auf, in welchem ein Biopolymer vorgesehen ist. Der Brennstoppbereich 103 kann an im Bereich eines dem Zündkopf 101 gegenüberliegenden Endes des Streichholzes 100 vorgesehen sein. Durch den Brennstoppbereich 103 wird ein brennbarer Bereich 102 des Streichholzes von einem nicht brennbaren Griffbereich 104 des Streichholzes 100 getrennt, in welchem das Streichholz 100 gefahrlos angefasst werden kann. Optional kann sich der Brennstoppbereich 103 bis an das dem Zündkopf 101 gegenüberliegende Ende des Streichholzes 100 erstrecken.

**Figur 19** zweigt einen Duftkörper 401. Der Duftkörper 401 ist nach Art einer Duftperle ausgebildet. Der Duftkörper 401 weist einen Duftstoff auf, der an die Umgebungsluft abgegeben werden kann. Ferner weist der Duftkörper 401 ein Biopolymer auf, so dass ein unerwünschtes Brennen des Duftkörpers verhindert wird. Der Duftkörper 401 ist in einem Aufnahmegefäß 400, beispielsweise einer Schale, angeordnet. Der Duftkörper 401 wird erwärmt, um den Duftstoff abzugeben. Bei einem Erwärmen des Duftkörpers 401 mit offenem Feuer, beispielsweise mit einer Kerze, kann ein unerwünschtes Brennen des Duftkörpers 401 verhindert werden.

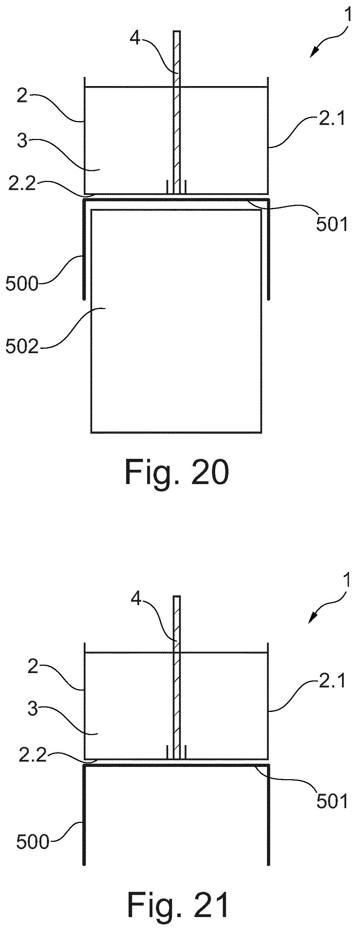

**Figur 20** zeigt eine Kerze 1. Hier ist das Kunststoffelement 2 als Mulde ausgebildet, in welcher das brennbare Material 3 aufgenommen werden kann. Das Kunststoffelement 2 weist bevorzugt eine kreisförmige Grundfläche auf. Das Kunststoffelement 2 weist einen Bodenbereich 2.2 und eine mit dem Bodenbereich 2.2 verbundene Seitenwand 2.1 auf. Der Bodenbereich 2.2 ist mit einer Auflagefläche 501 des Halteelements 500 verbunden, insbesondere verklebt, verklemmt oder versiegelt. Das Halteelement 500 ist so bemessen und geformt, dass es über eine Vorrichtung 502 zum Halten einer Kerze gestülpt werden kann. Das Kunststoffelement 2 und das Halteelement 500 sind aus demselben Material gebildet. Das bedeutet, dass sowohl das Kunststoffelement 2 als auch das Haltelement 500 ein Biopolymer enthalten. Über den Docht 4 kann brennbares Material 3 aus dem Inneren des Kunststoffelementes 2 verbrannt werden.

**Figur 21** zeigt ein weiteres Ausführungsbeispiel einer Kerze 1. Hier ist das Kunststoffelement 2 als Mulde ausgebildet, in welcher das brennbare Material 3 aufgenommen werden kann. Das Kunststoffelement 2 weist bevorzugt eine kreisförmige Grundfläche auf. Das Kunststoffelement 2 weist einen Bodenbereich 2.2 und eine mit dem Bodenbereich verbundene Seitenwand 2.1 auf. Der Bodenbereich 2.2 ist mit der Auflagefläche 501 des Halteelements 500 verbunden, insbesondere verklebt, verklemmt oder versiegelt. Das Halteelement 500 ist so bemessen und geformt, dass es als Sockel der Kerze genutzt werden kann. Das Kunststoffelement 2 und das Halteelement 500 sind aus demselben Material gebildet.

Das bedeutet, dass sowohl das Kunststoffelement 2 als auch das Haltelement 500 ein Biopolymer enthalten. Über den Docht 4 kann brennbares Material 3 aus dem Inneren des Kunststoffelementes 2 verbrannt werden.

### Bezugszeichenliste:

- 1: Kerze
- 2: Kunststoffelement
- 2.1: Seitenwand
- 2.2: Bodenbereich
- 2.3: Auflagefläche
- 2.4: Einbuchtung
- 2.4.1: Seitenwand
- 2.5: Rand
- 2.6: Form- und/oder Kraftschlussmittel
- 3: brennbares Material
- 4: Docht
- 5: Überzug
- 6: Gießform
- 7: Kunststoffelement, Dochthalter
- 8: Einlegeplättchen
- 9: Form

- 15: Verbindungsbereich
- 16: Deckel

- 100: Streichholz
- 101: Zündkopf
- 102: brennbarer Bereich
- 103: Brennstoppbereich
- 104: Griffbereich

- 200: Herstellungsvorrichtung
- 201: Auftragsvorrichtung
- 202: Biopolymer
- 203: Brennstoppbereich
- 204: Vorrichtung zur Herstellung einer Kerze

- 400: Aufnahmegefäß
- 401: Duftkörper

- 500: Halteelement
- 501: Auflagefläche des Halteelements
- 502: Vorrichtung zum Halten einer Kerze

## Patentansprüche

1. Verfahren zur Herstellung einer Kerze (1) aufweisend ein brennbares Material (3) und einen Docht (4) und ein becherartiges Gefäß (2), wobei das becherartige Gefäß (2) zumindest teilweise aus einem Biopolymer basierend auf Gelatine gebildet wird, **dadurch gekennzeichnet, dass**
- eine Form (9) des herzustellenden becherartigen Gefäßes (2) ein- oder mehrmals in das flüssige Biopolymer basierend auf Gelatine eingetaucht wird, bis eine gewünschte Materialstärke erreicht wird.

2. Verfahren nach Anspruch 1, **dadurch gekennzeichnet, dass** die Form (9) eine Tauchform des becherartigen Gefäßes (2) ist, wobei die Tauchform eine Außenkontur mit einem Tauchbereich aufweist, welcher in das flüssige Biopolymer eingetaucht wird, wobei der Tauchbereich bevorzugt einem Negativ der Innenkontur des herzustellenden becherartigen Gefäßes (2) entspricht.

3. Verfahren nach Anspruch 2, **dadurch gekennzeichnet, dass** die Form (9) in einem weiteren Schritt aus aus dem flüssigen Biopolymer entnommen wird und eine Schicht des Biopolymers, die die Außenkontur der Form überzieht, erstarrt und getrocknet wird.

4. Verfahren nach Anspruch 3, **dadurch gekennzeichnet, dass** die überzogene Form (9) während des Erstarrens und/oder Trocknens in einer oder mehreren Dimensionen gesteuert wird, um die Ausgestaltung des becherartigen Gefäßes (2), insbesondere die Form und/oder die Dicke gezielt einzustellen.

5. Verfahren nach einem der vorhergehenden Ansprüche, **dadurch gekennzeichnet, dass** das erstarrte becherartige Gefäß (2) mit einem brennbaren Material (3) und/oder mit einem Docht (4) verbunden wird.

6. Verfahren nach einem der vorhergehenden Ansprüche, **dadurch gekennzeichnet, dass** die Form (9) durch das brennbare Material (3) der Kerze gebildet wird.

7. Verfahren nach Anspruch 6, **dadurch gekennzeichnet, dass** die durch das das brennbare Material (3) gebildete Form (9) teilweise in das flüssige Biopolymer eingetaucht wird.

8. Verfahren nach Anspruch 6, **dadurch gekennzeichnet, dass** die durch das das brennbare Material (3) gebildete Form (9) vollständig in das flüssige Biopolymer eingetaucht wird.

9. Verfahren nach einem der vorhergehenden Ansprüche, **dadurch gekennzeichnet, dass** ein Docht (4) der Kerze beim Eintauchen des brennbaren Materials (3) in das flüssige Biopolymer bereits mit dem brennbaren Material (3) verbunden ist.

10. Verfahren nach Anspruch 9, **dadurch gekennzeichnet, dass** das brennbare Material (3) beim Eintauchen durch den Docht (4) gehalten wird, und das brennbare Material (3) mittels des Dochts (4) aus dem flüssigen Biopolymer herausgezogen wird.

## Claims

1. Method of manufacturing a candle (1) comprising a combustible material (3) and a wick (4) and a cup-like vessel (2), wherein the cup-like vessel (2) is formed at least in part from a biopolymer based on gelatine,
**characterized in that**
- a mould (9) of the cup-like vessel (2) to be produced is dipped one or more times into the liquid biopolymer based on gelatine until a desired material thickness is achieved.

2. Method according to claim 1, **characterized in that** the mould (9) is a dipping mould of the cup-like vessel (2), the dipping mould having an outer contour with a dipping region which is dipped into the liquid biopolymer, the dipping region preferably corresponding to a negative of the inner contour of the cup-like vessel (2) to be produced.

3. Method according to claim 2, **characterized in that** the mould (9) is removed from the liquid biopolymer in a further step and a layer of the biopolymer which covers the outer contour of the mould is solidified and dried.

4. Method according to claim 3, **characterized in that** the coated mould (9) is controlled in one or more dimensions during solidification and/or drying in order to control the configuration of the cup-like vessel (2), in particular to specifically adjust the shape and/or thickness.

5. Method according to one of the preceding claims, **characterized in that** the solidified cup-like vessel (2) is connected to a combustible material (3) and/or to a wick (4).

6. Method according to one of the preceding claims, **characterized in that** the mould (9) is formed by the combustible material (3) of the candle.

7. Method according to claim 6, **characterized in that** the mould (9) formed by the combustible material (3) is partially immersed in the liquid biopolymer.

8. Method according to claim 6, **characterized in that** the mould (9) formed by the combustible material (3) is completely immersed in the liquid biopolymer.

9. Method according to one of the preceding claims, **characterized in that** a wick (4) of the candle is already bonded to the combustible material (3) when the combustible material (3) is immersed in the liquid biopolymer.

10. Method according to claim 9, **characterized in that** the combustible material (3) is held by the wick (4) during immersion, and the combustible material (3) is pulled out of the liquid biopolymer by means of the wick (4).

## Revendications

1. Procédé de fabrication d'une bougie (1) présentant un matériau combustible (3) et une mèche (4) et un récipient en forme de gobelet (2), le récipient en forme de gobelet (2) étant formé au moins partiellement d'un biopolymère à base de gélatine, **caractérisé en ce que**
- un moule (9) du récipient en forme de gobelet (2) à fabriquer est immergé une ou plusieurs fois dans le biopolymère liquide à base de gélatine jusqu'à ce qu'une épaisseur de matériau souhaitée soit atteinte.

2. Procédé selon la revendication 1, **caractérisé en ce que** le moule (9) est un moule à immersion du récipient en forme de gobelet (2), le moule à immersion présentant un contour extérieur avec une zone d'immersion qui est immergée dans le biopolymère liquide, la zone d'immersion correspondant de préférence à un négatif du contour intérieur du récipient en forme de gobelet (2) à fabriquer.

3. Procédé selon la revendication 2, **caractérisé en ce que**, dans une étape ultérieure, le moule (9) est retiré du biopolymère liquide et une couche du biopolymère qui recouvre le contour extérieur du moule est solidifiée et séchée.

4. Procédé selon la revendication 3, **caractérisé en ce que** le moule revêtu (9) est commandé dans une ou plusieurs dimensions pendant la solidification et/ou le séchage, afin d'ajuster de manière ciblée la configuration du récipient en forme de gobelet (2), notamment la forme et/ou l'épaisseur.

5. Procédé selon l'une quelconque des revendications précédentes, **caractérisé en ce que** le récipient en forme de gobelet (2) solidifié est relié à un matériau combustible (3) et/ou à une mèche (4).

6. Procédé selon l'une quelconque des revendications précédentes, **caractérisé en ce que** le moule (9) est formé par le matériau combustible (3) de la bougie.

7. Procédé selon la revendication 6, **caractérisé en ce que** le moule (9) formé par le matériau combustible (3) est partiellement immergé dans le biopolymère liquide.

8. Procédé selon la revendication 6, **caractérisé en ce que** le moule (9) formé par le matériau combustible (3) est entièrement immergé dans le biopolymère liquide.

9. Procédé selon l'une quelconque des revendications précédentes, **caractérisé en ce qu'**une mèche (4) de la bougie est déjà liée au matériau combustible (3) lors de l'immersion du matériau combustible (3) dans le biopolymère liquide.

10. Procédé selon la revendication 9, **caractérisé en ce que** le matériau combustible (3) est maintenu par la mèche (4) lors de l'immersion, et le matériau combustible (3) est retiré du biopolymère liquide au moyen de la mèche (4) .
